# EUROPEAN PATENT SPECIFICATION

(11) **EP 0 532 153 B1**
(45) Date of publication and mention of the grant of the patent: **10.04.1996**
(21) Application number: 92304981.1
(22) Date of filing: 01.06.1992
(51) Int. Cl.: C07C 5/27

(54) **Isomerisation of hydrocarbons with solid superacid catalyst at supercritical or near critical conditions**
Isomerisierung von Kohlenwasserstoffen mit festen supersauren Katalysatoren unter überkritischen oder fast kritischen Zuständen
Isomérisation d'hydrocarbures avec catalyseurs solides suracides sous conditions surcritiques ou presque critiques

(30) Priority: 03.06.1991 US 709048
(43) Date of publication of application: 17.03.1993
(73) Proprietor: SUN COMPANY, INC. (R&M), Philadelphia, PA 19103-1699 (US)
(72) Inventor: Bhinde, Manoj V., Chadds Ford PA 19317 (US); Hsu, Chao-Yang, Media PA 19063 (US)
(74) Representative: Lewin, John Harvey

(56) References cited:
- US-A- 2 316 248
- US-A- 3 946 088
- US-A- 4 956 519
- DATABASE WPI Week 9028, Derwent Publications Ltd., London, GB; AN 90-213177 & JP-A-02 142 739 (AGENCY OF IND SCI TECH)

## Description

This invention relates to a novel process for isomerizing of paraffins using superacid catalysts under supercritical or near critical conditions. It is known that solid acids such as silica, alumina, zeolites, mordenite, or silica-alumina can be used as a heterogeneous catalyst for isomerization of linear paraffins to branched ones. It is also known that the use of these catalysts usually requires high reaction temperatures which cause catalyst deactivation due to the formation of coke deposits on the surface of the catalysts. Hence the catalyst activities are greatly reduced and the catalyst lives are shortened. Many approaches have been taken to eliminate coke formation problems: (1) add other ingredient(s) to the catalyst formulation to prevent coke formation, (2) use Pt or Pd in the catalyst composition and use hydrogen to remove the coke, or (3) operate the reaction under supercritical conditions so that the coke is dissolved in the supercritical paraffin fluids. The addition of other ingredients, especially a base metal such as Na or K, is effective for preventing coke formation, but the catalyst activities are reduced due to the decrease in catalyst acidities. Although it is quite effective to use Pt or Pd for hydrogenation of coke, the use of these precious metals increases the catalyst costs. Operating the isomerization under supercritical conditions has been demonstrated recently to be viable for maintaining the catalyst activities and lives. However, the use of the conventional solid acid catalysts such as silica, alumina, zeolites, mordenite or silica-alumina at supercritical conditions has the disadvantage of requiring high reaction temperatures and thus high reaction pressures are needed to stabilize the catalysts. For example, in recent publications (Sako, et. al., Sekiyu Gakkashi, Vol. 33,, Pages 92-98, (1990); and Japanese Patent 295801 (1988)) the authors reported that the stability of Y-zeolite catalyst in n-pentane isomerization could be maintained only under a reaction pressure of 40 MPa (6000 psi), and the reaction temperature was 587.2° K (314.2° C). Moreover, under such severe reaction conditions, the isopentane yield was well below 35%. Thus, supercritical processes employing the aforementioned conventional catalysts are unfeasible for commercial application.

Various solid superacids have been reported as very active paraffin isomerization catalysts. The use of a superacid catalyst has the advantage of lower reaction temperatures than are required with other isomerization catalysts and thus gives more thermodynamically favored branched paraffins and less light hydrocarbons. Due to its high activity, however, the superacid catalysts also could be easily deactivated either by the presence of trace amount of impurities in the feed or by the heavy polymers or coke or coke precursor produced in the reaction.

According to the present invention, it has been discovered that the disadvantages of the prior use of superacids in isomerization of paraffins are overcome by the use of supercritical or near critical conditions of temperature and pressure in combination with superacid catalysts. The life of the superacid catalyst is greatly extended when the isomerization is conducted under supercritical or near critical conditions, beyond the life obtained at conventional conditions. Supercritical or near critical conditions refer to temperatures at least 0.8 times the critical temperature and pressures at least as high as the critical pressure, of the substance involved.

The catalyst used according to the invention is a solid, strongly acidic material. Solid, very strongly acidic materials suitable for catalyzing hydrocarbon reactions, for example the isomerization of n-butane and alkylation, have been prepared in the prior art by treatment of zirconium oxides with sulfate ion or sulfuric acid, for example 1N sulfuric acid, and calcining the product at 500°C for three hours, as disclosed in Hino et al "Reactions of Butane and Isobutane Catalyzed by Zirconium Oxide Treated With Sulfate Ion", Journal of the American Chemical Society, Oct. 10, 1979, pages 3469-41. Solid superacids suitable for catalyzing skeletal isomerizations of butane and isobutane have been prepared by exposing H₄TiO₄ to 1N sulfuric acid and calcining in air at 500°C, as disclosed in Hino et al, "Reactions of Butane and Isobutane Catalyzed by Titanium Oxide Treated with Sulphate Ion", J.S.C. Chem Comm., 1979, pages 1148-9. Hino et al, "Synthesis of Solid Superacid Catalyst with Acid Strength of Hₒ = -16" disclose a preparation similar to that in the first Hino et al reference above (pages 3469-41), wherein Zr(OH)₄ obtained from different sources was calcined at temperatures up to 650°C, and found suitable for reactions of butane in a recirculation reactor at 25°C.

In Ito et al Japanese Patent No. 61-242641, solid acid catalysts for butane alkylation are prepared by impregnating sulfate-containing materials and rare earth metals or their compounds or supports consisting of Group IV metal hydroxides or oxides, followed by calcination and stabilization. Powdered Zr(OH)₄ supports were impregnated with lanthanum nitrate, dried, calcined at 300°C, treated with sulfuric acid, dried and calcined at 550°C for 3 hours.

In Japanese patent publication 87-344276/49, a solid superacid catalyst was prepared by impregnating a carrier comprising the hydroxide or oxide of a Group III or Group IV metal with a Group VIII metal (the abstract refers to Group VII, but the examples given are of Group VIII metals), for use in producing lower paraffin hydrocarbons from shale oil.

In Chemical Week, Nov. 25, 1987, the treatment of zirconium, titanium and iron oxides with sulfuric acids to produce "sulfated" inorganic oxides that show superior catalytic activity for alkylation of ortho-xylene by styrene, is disclosed.

In Baba et al Japanese Patent No. 61-2633932, Nov. 21, 1986, filed May 17, 1985, hydrocarbons are isomerized at reaction temperature below 400°C using a catalyst obtained by impregnating Group VIII metals, e.g. nickel, platinum, ruthenium, rhodium, palladium, osmium or iridium, and sulfate ion or precursor thereof in a carrier made of Group IV metals, e.g. titanium, zirconium, hafnium, silicon, germanium or tin, and/or hydroxide or oxide of Group III metals, e.g. aluminum, gallium, indium and thallium, and stabilizing by roasting at 450°-800°C for 5 to 16 hours.

In Veda et al Japanese Patent No. 62-246993, filed Apr. 2, 1986, paraffin hydrocarbons are thermally cracked at 150°-350°C and over 50 atmospheres hydrogen pressure in the presence of a solid, ultra strongly acidic catalyst. Said catalyst is usually made by treating hydroxides or impregnating a Group VIII metal, e.g. nickel, platinum, ruthenium, rhodium, palladium, osmium or iridium, on a supporting body of a hydroxide or oxide of Group III or Group IV metals, e.g. titanium, zirconium, silicon, germanium, tin, aluminum, gallium or indium, followed by treating with sulfuric acid and roasting to stabilize the catalyst.

The latter two references indicate that addition of Group VIII metal improves the catalytic activities of the solid superacids and that these solid superacids are suitable for isomerization of alkanes and xylenes, and cracking of shale oil or coal to light paraffins.

A solid superacid catalyst reported by K. Arata et al, J. Amer. Chem. Soc., 101, 6439 (1979), a sulfuric acid treated zirconium oxide, isomerized n-butane at 100° to 250°C, but the n-butane isomerization below 100°C is negligible.

In U. S. Patents 4,918,041 and 4,956,519, Hollstein et al, a sulfated and calcined catalyst contains (1) oxide or hydroxide of Group III or Group IV, e.g. zirconium, element, (2) oxide or hydroxide of Group V, Group VI or Group VII, e.g. manganese, metal and (3) oxide or hydroxide of Group VIII, e.g. iron, metal. The catalyst is disclosed as useful in the isomerization of normal alkanes having 4 to 7 carbon atoms per molecule, in alkylation of alkanes and alkylation of aromatics, dehydrogenation or partial oxidation of hydrocarbons and the conversion of alkenes and alcohols to ethers such as methyl tertiary butyl ether.

In Wen et al, "Hydroisomerization and Hydrocracking of n-Heptane and n-Hexadecane on Solid Superacids", Energy & Fuels, 1990, 4, 372-379, n-heptane and n-hexadecane are hydroisomerized and hydrocracked on a platinum-doped solid superacid, Pt/ZrO₂/SO₄²⁻, at 130-170°C, 300 psig initial hydrogen pressure and 15-75 minutes reaction time.
Pt/HfO₂/SO₄⁻² and Pt/ZrO₂/HfO₂/SO₄⁻² catalysts are also prepared and used in the reactions.

In Baba et al European Patent Application 85-306434.3 filed September 10, 1985, published March 19, 1986, publication number 0 174 836, solid strong acid catalysts in which sulfate (SO₄) and Group VIII metals are supported on hydroxides or oxides of Group III and/or Group IV metals, are disclosed as catalysts useful for hydrocarbon reactions such as isomerization of paraffinic hydrocarbons, isomerization of cyclic compounds and isomerization of aromatic compounds, cracking of n-pentane, and alkylation of isobutane and 2-butene.

In Hatakeyama et al Japanese Kokai Patent SHO 61-183230, August 15, 1986, from application No. SHO 60-23610 filed February 12, 1985, fractions rich in 2,2,3-trimethylpentane are produced by alkylation of C₄ hydrocarbons using a solid zirconia catalyst having acid strength (Hₒ) greater than -10.6, obtained by contacting zirconium hydroxide or oxide with a solution containing sulfate root.

In Japanese patent J01245853, issued to Keishitsu Ryubun Shi October 2, 1989, as reported in Derwent On-line Abstract no. 89-329274/45, a solid catalyst used for isobutane alkylation with olefin is disclosed, with a support material made of Group III and/or Group IV metal hydroxides or oxides, and containing Group IIb, Va, VIa, VIIa metals or its compounds, and SO₄ salts or SO₄-containing precursors. Examples of Group III metals given are aluminum, gallium, indium or thallium; of Group IV metals, titanium, zirconium, hafnium, silicon, germanium or tin; of Group IIb metals, zinc, cadmium or mercury; of Group Va (sic) metals, vanadium, niobium or tantalum; of Group VIa (sic) metals manganese or rhenium.

In Ito et al Japanese Kokai Patent, SHO 61-242641, October 28, 1956, application SHO 60-84515 filed April 22, 1985, a solid acidic catalyst for alkylation of isoparaffin with olefin is disclosed. The catalyst is obtained by adding a rare earth element or its compounds, and sulfate root or its precursor to a supporting member made of hydroxide or oxide of Group IV metals, followed by sintering at 400-800°C for stabilization. Hydroxide or oxide of at least one type of metals chosen from titanium, zirconium, hafnium, silicon, germanium and tin is used, and particularly hydroxide or oxide of zirconium or titanium is preferred. Tantalum and cerium or their compounds are disclosed as most desirable rare earths; praseodymium, neodymium, samarium and gadolinium are also disclosed.

The process of the invention, employing superacid catalysts at supercritical or near critical conditions, has unpredictable advantages over the use of superacid catalysts under conventional (not supercritical or near critical) conditions, as noted above, and also over the use of conventional catalysts (not superacids) at supercritical or near critical conditions.

Preferred superacids for use according to the invention are the solid acids which are known to have acidities stronger than 100% H₂SO₄ (i.e., Hₒ <-12). Examples of the solid superacids are sulfated zirconia, sulfated titania, sulfated iron oxide, sulfated zirconia containing one or more metals, sulfated titania and one or more metals, sulfated iron oxide and one or more other metals, halogenated alumina (such as fluorinated Al₂O₃, etc.), and a mixture of tungstate oxide and zirconia calcined at 800°C, etc. Other types of solid superacids are strong Lewis acids such as SbF₅, SbCl₅, SbF₅/HF, on a solid support such as silica, alumina or zirconia or combinations thereof.

The reaction temperatures range in reactions according to the invention from the critical temperature of the hydrocarbon feedstock, e.g. paraffinic hydrocarbons, to the highest acceptable temperatures which will give economically feasible yields of isoparaffin product. Typical reaction temperatures are in the range from 120°C to 300°C, depending upon the critical temperature of the feedstock. Preferably, the temperature is in the range from 0.8 to 1.2 times the critical temperature of the feedstock because lower temperatures result in lower activity for the desired conversion, and higher temperatures result in undesirable amounts of byproducts. The minimum reaction pressures for such reactions are the critical pressures of the paraffins. Preferred pressures are in the range from 500 psig to 2500 psig (3445 to 17225 KPa), again depending on the nature of the feedstock. Preferably the pressure is 2 to 3 times the critical pressure of the feedstock because lower pressures may have an adverse effect on catalyst life, and higher pressures undesirably affect equipment costs.

Preferably, the process is conducted at a temperature which is in the range from 0.8 to 1.2 times the higher of the critical temperatures of the principal reactant and the principal reaction product and the pressure of the isomerization is 2 to 3 times the higher of the critical pressures of said reactant and reaction product. The principal reactant, if there is more than one, is the reactant present in the largest proportion in the reactant material. The principal reaction product is the reaction product present in the largest proportion in the reaction product material.

Alternatively, the temperature and pressure of the reaction are in the range from 0.8 to 1.2 times the highest critical temperature and highest critical pressure respectively, of any reactant or reaction product which is present, in the reactant material or reaction product material respectively, in a proportion greater than ten percent of the respective materials.

Superacids are compounds which have acidity stronger than concentrated sulfuric acid (Hₒ = -12). Solid superacids are superacids which are solid at normal atmospheric temperatures and pressures.

The following examples illustrate the invention:

### Example 1

A sulfated zirconia containing 1.5% Fe and 0.5% Mn by weight was prepared by the following procedure:
Zirconium hydroxide is made by the hydrolysis of a zirconium salt solution (Solution A, 500 g. of zirconium carbonate in 300 g of conc. nitric acid and diluted to one liter) with ammonium hydroxide (Solution B, 370 g of conc. ammonium hydroxide in eight liter of water). Solutions A and B were simultaneously added to a reactor with stirring and heating such that the pH of the combined mixture was maintained between 7.0 to 8.0, and the temperature was held at 50-55°C until Solution A was completely used. After completion of the addition, the reaction mixture was stirred for an additional two hours and then allowed to cool with settling overnight. The solid hydroxide was separated and washed with water until the nitrate ion content in the wash water was less than 50 ppm. The solid was filtered and dried at 150°C overnight. Desirable particle size was obtained by grinding and sieving the dried zirconium hydroxide. Ammonium sulfate (5.52g.) was dissolved in sufficient amount of water and impregnated onto zirconium hydroxide (100 g). The amount of water required for the impregnation was determined by the state of "incipient wetness" for the weight of zirconium hydroxide. The treated hydroxide was then dried at 150°C and then calcined at 725°C for one hour. The finished catalyst, sulfated zirconia, contained 4.0% of sulfate ion. The acidity of the sulfated zirconia was reported by Hino and Arata to be Hₒ <-16 (J.C.S. Chem. Comm. 851, (1980)).

One or more transition metals on sulfated zirconia catalysts were made by impregnating the metal salts either on dried zirconium hydroxide or on sulfated hydroxide. The following procedures illustrate the preparation of a solid superacid, used in this invention, from dried zirconium hydroxide which contains 1.5% of Fe, 0.5% of Mn, and 4.0% of SO₄=: A solution consisting of 5.52g. of ammonium sulfate, 10.84g. of iron nitrate, and 2.28g. of manganese nitrate was prepared by dissolving the respective salts in suitable amount of water and impregnated onto dried zirconium hydroxide by incipient wetness. After drying the catalyst was calcined at 725°C for one hour.

The catalyst thus prepared is tested in a fixed bed reactor for isomerization of n-butane under the following reaction conditions: Temperature = 161°C, Pressure = 1500 psig (10 335 kPa), catalyst volume = 50 cc, butane feed rate = 100 cc/hr. The critical temperature and critical pressure of n-butane are about 152°C and about 550 psig (3789 kPa), respectively. After 70 hrs. of reaction time, the product stream is analyzed using gas chromatography. The product composition is shown in Run 1 of Table I. After 200 hours of reaction time, the product stream is again analyzed and the results are shown in Run 2 of Table 1.

Comparison of the results in this example with those in the Sako et al and Japanese patent 295801 references above show that good results are obtained at temperature and pressure of 161°C and 1500 psig (10 335 kPa) for butane isomerization, using superacid catalyst at supercritical conditions, as compared to 314°C and 6000 psig (41 340 kPa), in the prior art use of conventional catalyst for pentane isomerization at supercritical conditions. Also, the operation according to the invention has the benefit of supercritical conditions in protecting the catalyst surfaces from deactivating deposits.

### Example 2

The same procedures as described in Example 1, except that the butane feed rate is 50 cc/hr., are used to test the catalyst, sulfated zirconia containing 1.5% Fe and 0.5% Mn. Analysis results of the product streams after reaction times of 200 hrs., 300 hrs., and 400 hrs. are shown in Table II.

**TABLE I**

| Product Distribution of Butane Isomerization Catalyzed by Solid Superacid and Under Supercritical Conditions | | |
|---|---|---|
| Reaction Conditions | Run 1 | Run 2 |
| Reaction Time (hr.) | 70 | 200 |
| Reaction Temp. (°C) | 161 | 162 |
| Reaction Press. (psi) | 1500 (10 335 kPa) | 1500 (10 335 kPa) |
| Butane Feed (cc/hr) | 100 | 100 |
| Catalyst Vol. (cc) | 50 | 50 |

| Product Compositions (% wt.) | | |
|---|---|---|
| Propane | 4.3 | 4.0 |
| Isobutane | 43.8 | 42.7 |
| Butane | 45.8 | 47.2 |
| Isopentane | 4.3 | 4.1 |
| Pentane | 1.3 | 1.3 |
| Others | 0.5 | 0.7 |

**TABLE II**

| Product Distribution of Butane Isomerization Catalyzed by Solid Superacid and Under Supercritical Conditions | | | |
|---|---|---|---|
| Reaction Time (hr.) | 200 | 300 | 400 |
| Product Distribution | | | |
| Propane | 6.7 | 6.3 | 6.2 |
| Isobutane | 45.9 | 45.9 | 45.8 |
| Butane | 38.8 | 39.7 | 40.0 |
| Isopentane | 6.2 | 5.9 | 5.8 |
| Pentane | 2.0 | 1.9 | 1.9 |
| Others | 0.4 | 0.3 | 0.3 |

## Claims

1. Process for isomerization of a straight chain paraffin having 4 to 24 carbon atoms per molecule which comprises contacting said paraffin with a solid superacid catalyst with reactant and/or product in a supercritical or near critical state.

2. Process according to Claim 1 wherein the temperature of the isomerization is in the range from 0.8 to 1.2 times the higher of the critical temperatures of the principal reactant and the principal reaction product, and the pressure of the isomerization is in the range from 2 to 3 times the higher of the critical pressures of said reactant and reaction product.

3. Process according to claim 1 wherein the acidity of the catalyst is stronger than Hₒ = -12.

4. Process according to claim 1 wherein the solid superacid comprises sulfated zirconia.

5. Process according to claim 1 wherein the solid superacid comprises sulfated zirconia containing one or more metal compounds.

6. Process according to claim 1 wherein reactant and product are in a near critical state.

## Patentansprüche

1. Verfahren zur Isomerisierung eines geradekettigen Paraffins mit 4 bis 24 Kohlenstoffatomen pro Molekül, welches das Kontaktieren des Paraffins mit einem festen Supersäure-Katalysator umfaßt, wobei der Reaktant und/oder das Produkt sich im überkritischen Zustand oder im nahezu kritischen Zustand befindet.

2. Verfahren nach Anspruch 1, wobei die Temperatur der Isomerisierung im Bereich des 0,8- bis 1,2-fachen der höheren der kritischen Temperaturen des Haupt-Reaktanten und des Haupt-Reaktionsprodukts liegt und der Druck der Isomerisierung im Bereich des 2- bis 3-fachen des höheren der kritischen Drücke des Reaktanten und des Reaktionsprodukts liegt.

3. Verfahren nach Anspruch 1, wobei die Azidität des Katalysators stärker ist als Hₒ = -12.

4. Verfahren nach Anspruch 1, wobei die feste Supersäure sulfatisiertes Zirconiumoxid umfaßt.

5. Verfahren nach Anspruch 1, wobei die feste Supersäure sulfatisiertes Zirconiumoxid umfaßt, das eine oder mehr Metallverbindungen enthält.

6. Verfahren nach Anspruch 1, wobei der Reaktant und das Produkt sich in nahezu kritischen Zustand befinden.

## Revendications

1. Procédé d'isomérisation d'une paraffine à chaîne droite possédant 4 à 24 atomes de carbone par molécule, caractérisé en ce que l'on met cette paraffine en contact avec un catalyseur du type superacide solide, avec le réactif et/ou le produit à l'état supercritique ou un état proche de l'état critique.

2. Procédé suivant la revendication 1, caractérisé en ce que la température de l'isomérisation atteint la plage de 0,8 à 1,2 fois la plus élevée des températures critiques du réactif principal et du produit de réaction principal et la pression de l'isomérisation atteint la plage de 2 à 3 fois la plus élevée des pressions critiques du réactif et du produit de réaction principal.

3. Procédé suivant la revendication 1, caractérisé en ce que l'acidité du catalyseur est supérieure à Hₒ = -12.

4. Procédé suivant la revendication 1, caractérisé en ce que le superacide solide est constitué de zircone sulfatée.

5. Procédé suivant la revendication 1, caractérisé en ce que le superacide solide est constitué de zircone sulfatée contenant un ou plusieurs composés de métaux.

6. Procédé suivant la revendication 1, caractérisé en ce que le réactif et le produit se trouvent dans un état proche de l'état critique.
